# EUROPÄISCHE PATENTSCHRIFT

(11) **EP 2 909 149 B1**
(45) Veröffentlichungstag und Bekanntmachung des Hinweises auf die Patenterteilung: **21.12.2016**
(21) Anmeldenummer: 13766069.2
(22) Anmeldetag: 23.09.2013
(51) Int. Cl.: C03C 17/00

(54) **STRAHLEREINHEIT ZUR ERZEUGUNG ULTRAVIOLETTER STRAHLUNG SOWIE VERFAHREN ZU DEREN HERSTELLUNG**
RADIATOR UNIT FOR GENERATING ULTRAVIOLET RADIATION AND METHOD FOR THE PRODUCTION THEREOF
UNITÉ DE PROJECTEUR POUR LA PRODUCTION DE RAYONNEMENT ULTRAVIOLET ET PROCÉDÉ POUR LA FABRICATION DE LADITE UNITÉ DE PROJECTEUR

(30) Priorität: 18.10.2012 DE 102012109930
(43) Veröffentlichungstag der Anmeldung: 26.08.2015
(73) Patentinhaber: Heraeus Noblelight GmbH, 63450 Hanau (DE)
(72) Erfinder: RUIZ, Hector Julian, 63450 Hanau (DE)
(74) Vertreter: Heraeus IP
(86) Internationale Anmeldenummer: PCT/EP2013/069718
(87) Internationale Veröffentlichungsnummer: WO 2014/060190

(56) Entgegenhaltungen:
- US-A1- 2008 038 458
- US-A1- 2009 075 093
- US-A1- 2009 145 855
- US-B2- 6 932 947

## Beschreibung

Die vorliegende Erfindung betrifft eine Strahlereinheit zur Erzeugung ultravioletter Strahlung, insbesondere zum Einsatz bei der Lebensmittelverarbeitung oder bei der Aufbereitung von Wasser, umfassend einen UV-Strahler mit einem Strahlerrohr aus Quarzglas oder einen von einem zylinderförmigen Hüllrohr aus Quarzglas umgebenen UV-Strahler mit einem Strahlerrohr aus Quarzglas.

Die vorliegende Erfindung betrifft weiterhin ein Verfahren zur Herstellung der Strahlereinheit.

### Stand der Technik

Mögliche Einsatzbereiche von Strahlereinheiten sind beispielsweise die Aufbereitung und Desinfektion von Wasser, die Reinigung und Entkeimung von Gasen oder Gasgemischen, insbesondere von Luft, sowie die Entkeimung von Oberflächen.

Derartige Strahlereinheiten umfassen einen UV-Strahler mit einem Strahlerrohr aus Quarzglas; sie werden beispielsweise in Wasseraufbereitungsanlagen, Lüftungssystemen, Abzugsvorrichtungen für Gase oder Luftaufbereitungsanlagen eingesetzt. Je nach Einsatzzweck kann eine solche Strahlereinheit beispielsweise zur Abtötung von Mikroorganismen, zur Beseitigung auftretender Gerüche oder zur Zersetzung von Verunreinigungen beitragen.

Häufig werden Strahlereinheiten bei der Verarbeitung von Lebensmitteln eingesetzt. Sie finden sowohl bei der industriellen Fertigung von Lebensmitteln, als auch in Großküchen oder im häuslichen Bereich Anwendung.

Bei der Verarbeitung von Lebensmitteln entstehen Koch- und Backdünste, die auch als Wrasen bezeichnet werden. Sie enthalten neben Wasserdampf eine Vielzahl von Schweb- und Geruchsstoffen, insbesondere Fette.

Um die bei der Verarbeitung der Lebensmittel auftretenden Gerüche zu neutralisieren (Desodorierung) und gleichzeitig eine Ablagerung von Schwebstoffen zu verringern, werden beispielsweise in Lüftungssystemen oder Abluftvorrichtungen neben mechanischen Filtern auch Strahlereinheiten zur Erzeugung ultravioletter Strahlung eingesetzt. Der Einsatz dieser UV-Strahlereinheit ermöglicht eine chemische Zersetzung von Geruchs- und Schwebstoffen.

Um eine effiziente Bestrahlung mit ultravioletter Strahlung zu gewährleisten, sind die Strahlereinheiten in der Regel so angeordnet, dass sie von den Wrasen umströmt werden. Während des Betriebs der Strahlereinheit können sich daher auf dem Strahlerrohr Staubpartikel oder Verunreinigungen, insbesondere Fettablagerungen, niederschlagen. Diese Verunreinigungen absorbieren die von dem UV-Strahler emittierte UV-Strahlung, so dass die Transparenz des Strahlerrohrs und damit die Effizienz der UV-Bestrahlung mit zunehmender Betriebsdauer abnehmen. Dies hat zur Folge, dass das Strahlerrohr regelmäßig gereinigt oder der Strahler ausgetauscht werden muss. Um eine Verschmutzung des Strahlerrohrs zu verringern, ist es bekannt, vor der Strahlereinheit mechanische Filter zu installieren. Allerdings führen auch diese Filter nur zur teilweisen Abtrennung von Verunreinigungen. Darüber hinaus setzt das Vorsehen mechanischer Filter einen regelmäßigen Austausch der Filter voraus und ist daher zeit- und kostenintensiv.

Darüber hinaus ist in vielen Strahlereinheiten, insbesondere bei solchen, die für die Aufbereitung von Flüssigkeiten eingesetzt werden, der UV-Strahler häufig dadurch vor Verschmutzung geschützt, dass er in einem Hüllrohr aus Quarzglas angeordnet ist. Durch das Hüllrohr wird das Strahlerrohr nicht unmittelbar von dem Fluid umströmt, so dass Ablagerungen von Verunreinigungen auf dem Strahlerrohr vermindert werden.

So ist beispielsweise ist aus der WO 2008/059503 A1 eine Anlage zur Entkeimung von Flüssigkeiten mittels ultravioletter Strahlung bekannt, die einen Strömungskanal aufweist. Innerhalb des Strömungskanals sind senkrecht zur Strömungsrichtung mehrere Strahler angeordnet, die von einem Hüllrohr umgeben sind.

Allerdings ist bei Einsatz eines Hüllrohres dieses selbst einer Verschmutzung ausgesetzt, so dass in Abhängigkeit vom Verschmutzungsgrad die Transmissionseigenschaften des Hüllrohres und damit die Effizienz der Strahlerleistung beeinträchtigt werden. Darüber hinaus können sich auf dem Hüllrohr Biofilme bilden, die ebenfalls eine Transmission ultravioletter Strahlung beeinträchtigen, so dass auch Hüllrohre in regelmäßigen Abständen aufwendig maschinell oder von Hand gereinigt werden müssen.

US 6 932 947 B2 beschreibt Strahlereinheiten zur Aufbearbeitung von Wasser.

### Technische Aufgabe

Der Erfindung liegt daher die Aufgabe zugrunde, eine Strahlereinheit zur Erzeugung optischer Strahlung anzugeben, die geeignet ist, über eine lange Betriebsdauer eine hohe Strahlungsleistung zu emittieren, und die darüber hinaus einfach und kostengünstig zu fertigen ist.

Weiterhin liegt der Erfindung die Aufgabe zugrunde, ein Verfahren zur Herstellung einer solchen Strahlereinheit anzugeben.

### Allgemeine Beschreibung der Erfindung

Hinsichtlich der Strahlereinheit wird diese Aufgabe ausgehend von einer Strahlereinheit zur Erzeugung ultravioletter Strahlung der eingangs genannten Gattung erfindungsgemäß dadurch gelöst, dass auf das Strahlerrohr und/oder das Hüllrohr eine schmutzabweisende Beschichtung aufgebracht ist, die unter Einsatz von Siliciumdioxid- oder Titandioxid-Nanopartikeln erzeugt ist.

Eine aus Siliciumdioxid- oder Titandioxid-Nanopartikeln erzeugte Beschichtung, weist insbesondere eine hohe Transparenz für ultraviolette Strahlung auf. Sie ist daher zur Beschichtung des Hüllrohres beziehungsweise Strahlerrohres geeignet. Eine solche Beschichtung beeinträchtigt die Strahlungsleistung des Strahlers nicht wesentlich.

Darüber hinaus kann eine solche Beschichtung aufgrund ihrer chemischen Eigenschaften dauerhaft auf eine Quarzglas-Oberfläche aufgebracht werden. Eine aus Siliciumdioxid- oder Titandioxid-Nanopartikeln erzeugte Beschichtung weist eine hohe UV-Stabilität, eine gute Abrasionsbeständigkeit und eine gute Temperaturbeständigkeit auf. Sie verfügt darüber hinaus über eine hohe chemische Stabilität.

Erfindungsgemäß ist die Beschichtung auf das Strahlerrohr und/oder ein das Strahlerrohr umgebendes Hüllrohr aufgebracht. Nachfolgend wird zur Vereinfachung der Beschreibung anstelle der Begriffe Strahlerrohr und Hüllrohr der Oberbegriff Rohr verwendet, wobei sich die zugehörige Beschreibung auf beide Varianten erstrecken soll.

Die Beschichtung bedeckt das Rohr vollständig oder teilweise. Vorzugsweise ist Beschichtung auf eine äußere Oberfläche des Rohres aufgebracht.

Aus Quarzglas gefertigte Rohre können eine leicht raue Oberfläche aufweisen, die eine Ablagerung von Schmutzpartikeln grundsätzlich begünstigt. Eine Beschichtung mit Nanopartikeln ist daher insbesondere geeignet, Unebenheiten der Quarzglasoberfläche auszufüllen. Durch das Aufbringen einer Beschichtung aus Nanopartikeln, wird die Oberflächenrauheit verringert, so dass eine glattere Oberfläche erhalten wird, an der Schmutzpartikel weniger haften.

Weiterhin tragen die physikalisch-chemischen Eigenschaften der Siliciumdioxid- oder Titandioxid-Nanopartikel dazu bei, die Adsorption und Ablagerung von Schmutzpartikeln zu unterbinden. So weist eine erfindungsgemäß beschichtete, verglichen mit einer unbeschichteten, Rohroberfläche eine höhere Hydrophilie auf, wordurch die Ablagerung von lipophilen Verunreinigungen erschwert wird.

Eine Strahlereinheit mit einem beschichteten Hüll- oder Strahlerrohr kann daher ohne Reinigung über einen langen Zeitraum bei hoher Strahlungsleistung betrieben werden. Durch die verlängerten Reinigungsintervalle wird einen einfacher und kostengünstiger Betrieb der Strahlereinheit ermöglicht.

In einer vorteilhaften Ausgestaltung der erfindungsgemäßen Strahlereinheit ist vorgesehen, dass die Beschichtung keine organischen Substanzen umfasst.

Die Beschichtung des Hüllkolbens oder des Strahlerrohrs ist während des Betriebs der Strahlereinheit einer dauerhaften Bestrahlung mit ultravioletter Strahlung ausgesetzt. Eine Bestrahlung organischer Substanzen mit ultravioletter Strahlung begünstigt allerdings deren Zersetzung und führt zu einer geringen Lebensdauer der Beschichtung. Eine Beschichtung hoher Lebensdauer wird erhalten, wenn die Beschichtung ausschließlich anorganische Substanzen umfasst.

In einer vorteilhaften Ausgestaltung der erfindungsgemäßen Strahlereinheit ist vorgesehen, dass die Beschichtung eine Oberfläche mit einer mittleren Rauheit Rₐ von weniger als 0,05 µm aufweist.

Die mittlere Rauheit Rₐ wird als Senkrechtmessgröße nach DIN EN ISO 4288:1988 bestimmt. Sie gibt den mittleren Abstand eines Messpunktes bezogen auf eine Mittellinie eines Oberflächenprofils an. Eine Oberfläche mit einer Rauheit von mehr als 0,05 µm ist nur begrenzt schmutzabweisend. Es hat sich daher bewährt, wenn die mittlere Rauheit Rₐ der beschichteten Oberfläche weniger als 0,05 µm beträgt.

Es hat sich als vorteilhaft erwiesen, wenn die Siliciumdioxid-Nanopartikel eine mittlere Teilchengröße im Bereich von 10 nm bis 75 nm aufweisen. Silicium-Nanopartikel mit einer mittleren Teilchengröße im Bereich von 10 nm bis 75 nm sind einfach und kostengünstig herzustellen. Sie sind insbesondere geeignet, Unebenheit auf einer Quarzglasoberfläche auszugleichen.

Es hat sich bewährt, wenn die Titandioxid-Nanopartikel eine mittlere Teilchengröße zwischen 10 nm und 80 nm aufweisen.

Titandioxid-Nanopartikel mit einer mittleren Teilchengröße zwischen 10 nm und 80 nm sind einfach und kostengünstig herstellbar. Die mittlere Teilchengröße der Nanopartikel beeinflusst die Oberflächenstruktur der Beschichtung. Eine Beschichtung mit Titandioxid-Nanopartikeln mit einer mittleren Teilchengröße von mehr als 80 nm weist eine vergleichsweise grobe Oberflächenstruktur auf. Nanopartikel mit einer mittleren Teilchengröße von weniger als 10 nm sind aufwendig zu verarbeiten.

Bei einer vorteilhaften Ausgestaltung der Strahlereinheit beträgt die mittlere Schichtdicke der Beschichtung zwischen 60 nm und 150 nm.

Die Schichtdicke der Beschichtung beeinflusst die Transmission des Strahlerbeziehungsweise Hüllrohres. Eine einheitliche Beschichtung mit einer mittleren Schichtdicke von weniger als 60 nm ist nur aufwendig herzustellen. Beschichtungen mit einer mittleren Schichtdicke von mehr als 150 nm können leicht Abblättern und weisen eine geringere Lebensdauer auf.

Es hat sich als günstig erwiesen, wenn das Strahlerrohr und/oder das Hüllrohr eine Oberfläche mit einer mittleren Rauheit Rₐ im Bereich zwischen 0,01 µm und 1 µm aufweist, auf die die Beschichtung aufgebracht ist.

Die Haftung der Beschichtung auf der Strahlerrohr-/Hüllrohr-Oberfläche wird durch die mittlere Rauheit der Oberfläche beeinflusst. Ein Strahlerrohr mit einer mittleren Rauheit Rₐ von weniger als 0,01 µm weist eine geringe Oberflächenstruktur auf und führt zu einer schlechteren Haftung der Beschichtung. Eine Oberfläche mit einer mittleren Rauheit Rₐ von mehr als 1 µm erfordert eine vergleichsweise große Schichtdicke der glättenden Beschichtung.

In einer weiteren bevorzugten Ausgestaltung ist vorgesehen, dass das Strahlerrohr eine Abstrahlfläche aufweist, die vollständig mit der Beschichtung versehen ist.

Bei einem Strahlerrohr mit einer vollständig beschichteten Abstrahlfläche ist die gesamte Abstrahlfläche schmutzabweisend. Ein solches Strahlerrohr trägt zu einer gleichmäßigen Strahlungsleistung des Strahlers über die gesamte Einsatzdauer des Strahlers bei.

Hinsichtlich des Herstellverfahrens wird diese Aufgabe ausgehend von einem Verfahren der eingangs genannten Gattung erfindungsgemäß dadurch gelöst, dass das Verfahren folgende Verfahrensschritte umfasst:
(a) Auftragen einer alkoholischen Dispersion von Siliciumdioxid- oder Titandioxid-Nanopartikeln auf die Außenwandung unter Bildung einer Dispersionsschicht, wobei die alkoholische Dispersion 20 Vol.-% bis 60 Vol.-% Ethanol umfasst, jeweils bezogen auf das Volumen der Dispersion,
(b) Aushärten der Dispersionsschicht unter Bildung der Beschichtung.

Zur Beschichtung wird auf die Außenwand des Strahlerrohres oder der Hüllrohres eine alkoholische Dispersion von Siliciumdioxid- oder Titandioxid-Nanopartikeln aufgetragen. Neben Ethanol in einem Konzentrationsbereich von 20 Vol.-% bis 60 Vol.-% kann die Dispersion andere flüchtige Lösungsmittel, beispielsweise Methanol, Isopropanol oder Mischungen davon enthalten. Ethanol weist eine gewisse Hydrophilie auf und ist darüber hinaus mit lipophilen Substanzen begrenzt mischbar. Darüber hinaus liegt der Siedepunkt von Ethanol bei 78 °C. Durch eine ethanolische Dispersion wird daher bereits ein Trocknen der Dispersion bei niedrigen Temperaturen, beispielsweise bei Raumtemperatur, ermöglicht. Nach dem Verdampfen des Lösungsmittels verketten sich die Siliciumdioxid-Nanopartikel zu einem dichten Netz. Titandioxid-Nanopartikel hingegen bilden hingegen eine Beschichtung aus vorrangig diskreten Titandioxid-Partikeln.

In einer bevorzugten Modifikation des Verfahrens ist vorgesehen, dass die alkoholische Dispersion 0,25 Vol.-% bis 1,5 Vol.-% 2-Butanon umfasst.

2-Butanon weist einen Siedepunkt von 80 °C auf und ist ein gutes Lösungsmittel für lipophile Substanzen. Ein Zusatz von 2-Butanon zum Dispersion erhöht die Lipophile des Dispersionsmittels.

### Ausführungsbeispiel

Nachfolgend wird die Erfindung anhand eines Ausführungsbeispiels und einer Zeichnung mit zwei Figuren näher beschrieben. Im Einzelnen zeigt in schematischer Darstellung:
- **Figur 1**: eine Ausführungsform der erfindungsgemäßen Strahlereinheit mit einem beschichteten Strahlerrohr in Seitenansicht, und
- **Figur 2**: eine zweite Ausführungsform der erfindungsgemäßen Strahlereinheit mit einem beschichteten Hüllrohr im Querschnitt.

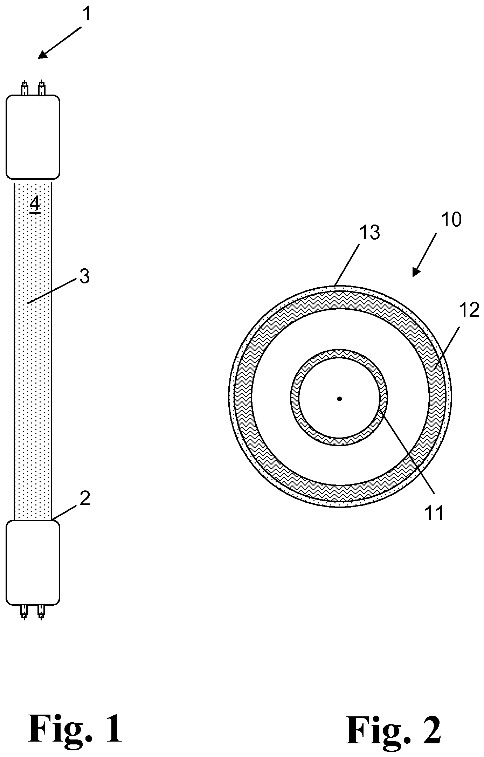

**Figur 1** zeigt schematisch eine Ausführungsform der erfindungsgemäßen Strahlereinheit zur Erzeugung ultravioletter Strahlung, der insgesamt die Bezugsziffer 1 zugeordnet ist. Die Strahlereinheit 1 ist zum Einsatz in Großküchen, insbesondere zur Reduktion auftretender Gerüche beziehungsweise zur Verminderung von Fettablagerungen geeignet. von Geruchs- und Schwebstoffen von Koch- und Backwrasen geeignet.

Die Strahlereinheit 1 umfasst einen UV-Strahler 2 mit einem Strahlerrohr 3 aus Quarzglas. Der UV-Strahler 2 zeichnet sich durch eine Nominal-Leistung von 500 W bei einem nominalen Lampenstrom von 2,5 A, eine Leuchtlänge von 1000 mm und einen Leuchtrohr-Außendurchmesser von 24 mm aus. Auf die Außenwandung des Strahlerrohrs 3 ist eine schmutzabweisende Beschichtung 4 aufgebracht, wobei die Beschichtung 4 die Abstrahlfläche des Strahlerrohrs 3 vollständig bedeckt. Die Beschichtung 4 ist frei von organischen Substanzen. Zur Erzeugung der Beschichtung auf der Außenwand des Strahlerrohrs 3 wird eine ethanolische Dispersion von Siliciumdioxid-Nanopartikeln folgender Zusammensetzung verwendet: 50 Vol.-% Ethanol, 49 Vol.-% Siliciumdioxid-Nanopartikel (mittlere Teilchengröße 50 nm), 1 Vol.-% 2-Butanon. Die ethanolische Dispersion wird auf die Außenwand des Strahlerrohrs 3 manuell aufgetragen, wobei die Außenwand eine mittlere Rauheit Rₐ von 0,25 µm aufweist. Die Dispersion kann alternativ auf die Außenwand auch aufgesprüht werden. Anschließend wird das Strahlerrohr 3 für 24 Stunden bei Raumtemperatur unter Ausbildung der Beschichtung 4 getrocknet. Das beschichtete Strahlerrohr 3 weist eine mittlere Rauheit Rₐ von 0,02 µm auf. Die Schichtdicke der Beschichtung 4 beträgt 120 nm.

In einer alternativen Ausführungsform der erfindungsgemäßen Strahlereinheit ist die Beschichtung 4 aus Titandioxid-Nanopartikeln mit einer mittleren Teilchengröße von 75 nm hergestellt.

**Figur 2** zeigt im Querschnitt eine zweite Ausführungsform der erfindungsgemäßen Strahlereinheit 10 mit einem zylinderförmigen Strahler 11, der von einem Hüllrohr 12 aus Quarzglas umgeben ist. Die Strahlereinheit 10 ist zum Einsatz in eine Wasseraufbereitungsanlage (nicht dargestellt) geeignet.

Auf das zylinderförmige Hüllrohr 12 ist eine unter Einsatz von Siliciumdioxid-Nanopartikeln erzeugte schmutzabweisende Beschichtung 13 aufgebracht. Die Oberfläche des beschichteten Strahlerrohrs weist eine mittlere Rauheit von 0,007 µm auf.

### Beispiel 1

Zu Vergleichszwecken wurde eine Küchenhaube mit einer erfindungsgemäßen Strahlereinheit gemäß Figur 1 und eine weitere Küchenhaube mit einer baugleichen, herkömmlichen Strahlereinheit betrieben. Anschließend wurde die Transparenz des Lampenkolbens visuell beurteilt. Die Ergebnisse dieser Untersuchungen sind in den nachfolgenden Tabellen zusammengefasst:

### Ergebnisse 1

| **Lampentyp** | **Betriebsdauer** | **Optische Prüfung** |
|---|---|---|
| Strahlerrohr mit halbseitiger Beschichtung | 3 Monate (900 Betriebsstunden) | Klares, durchsichtiges Strahlerrohr |
| Standard | 3 Monate (900 Betriebsstunden) | Milchig, trübes Strahlerrohr |

### Ergebnisse 2

| **Lampentyp** | **Betriebsdauer** | **Optische Prüfung** |
|---|---|---|
| Strahlerrohr mit halbseitiger Beschichtung | 7 Monate (2100 Betriebsstunden) | Klares, durchsichtiges Strahlerrohr, einige trüben Stellen |
| Standard | 7 Monate (2100 Betriebsstunden) | Milchig, trübes Strahlerrohr |

Strahlereinheiten mit einem beschichteten Strahlerrohr zeigen auch bei längerer Betriebsdauer keine oder nur geringfügige Schmutz-Ablagerungen. Die Strahlerrohre mit einer Beschichtung sind vielmehr auch nach über 2.000 Betriebsstunden klar und durchsichtig.

### Beispiel 2

Zu Vergleichszwecken wurde eine Quarzplatte teilweise mit Siliciumdioxid-Nanopartikel beschichtet und die Kontaktwinkel mit Wasser gemessen. Die Ergebnisse dieser Untersuchungen sind in den nachfolgenden Tabellen zusammengefasst:

| **Quarzplatte** | **Kontaktwinkel nach 1 Stunde (bei 120°C)** | **Kontaktwinkel nach 24 Stunden (bei 120°C)** |
|---|---|---|
| Beschichtet | 60° | 62° |
| Unbeschichtet | 25° | 30° |

## Patentansprüche

1. Strahlereinheit zur Erzeugung ultravioletter Strahlung, insbesondere zum Einsatz bei der Lebensmittelverarbeitung oder bei der Aufbereitung von Wasser, umfassend einen UV-Strahler mit einem Strahlerrohr aus Quarzglas oder einen von einem zylinderförmigen Hüllrohr aus Quarzglas umgebenen UV-Strahler mit einem Strahlerrohr aus Quarzglas, **dadurch gekennzeichnet, dass** auf das Strahlerrohr und/oder das Hüllrohr eine schmutzabweisende Beschichtung aufgebracht ist, die unter Einsatz von Siliciumdioxid- oder Titandioxid-Nanopartikeln erzeugt ist.

2. Strahlereinheit nach Anspruch 1, **dadurch gekennzeichnet, dass** die Beschichtung keine organischen Substanzen umfasst.

3. Strahlereinheit nach Anspruch 1 oder 2, **dadurch gekennzeichnet, dass** die Beschichtung eine Oberfläche mit einer mittleren Rauheit Ra von weniger als 0,05 µm aufweist.

4. Strahlereinheit nach einem der vorhergehenden Ansprüche, **dadurch gekennzeichnet, dass** die Siliciumdioxid-Nanopartikel eine mittlere Teilchengröße im Bereich von 1 nm bis 75 nm aufweisen.

5. Strahlereinheit nach einem der vorhergehenden Ansprüche, **dadurch gekennzeichnet, dass** die Titandioxid-Nanopartikel eine mittlere Teilchengröße zwischen 1 nm und 80 nm aufweisen.

6. Strahlereinheit nach einem der vorhergehenden Ansprüche, **dadurch gekennzeichnet, dass** die mittlere Schichtdicke der Beschichtung zwischen 60 nm und 150 nm beträgt.

7. Strahlereinheit nach einem der vorhergehenden Ansprüche, **dadurch gekennzeichnet, dass** das Strahlerrohr und/oder das Hüllrohr eine Oberflächeit einer mittleren Rauheit Rₐ im Bereich zwischen 0,01 µm und 1 µm aufweist, auf die die Beschichtung aufgebracht ist.

8. Strahlereinheit nach einem der vorhergehenden Ansprüche, **dadurch gekennzeichnet, dass** das Strahlerrohr eine Abstrahlfläche aufweist, die vollständig mit der Beschichtung versehen ist.

9. Verfahren zur Herstellung einer Strahlereinheit nach Anspruch 1 mit einem beschichteten Strahlerrohr aus Quarzglas und/oder einem beschichteten Hüllrohr aus Quarzglas durch Bereitstellen des Strahlerrohrs oder des Hüllrohrs und Erzeugen einer Beschichtung auf mindestens einem Teil der Außenwandung, umfassend folgende Verfahrensschritte:
(c) Auftragen einer alkoholischen Dispersion von Siliciumdioxid- oder Titandioxid-Nanopartikeln auf die Außenwandung unter Bildung einer Dispersionsschicht, wobei die alkoholische Dispersion 20 Vol.-% bis 60 Vol.-% Ethanol umfasst, jeweils bezogen auf das Volumen der Dispersion,
(d) Aushärten der Dispersionsschicht unter Bildung der Beschichtung.

10. Verfahren nach Anspruch 9, **dadurch gekennzeichnet, dass** die alkoholische Dispersion 0,25 Vol.-% bis 1,5 Vol.-% 2-Butanon umfasst.

## Claims

1. Emitter unit for generating ultraviolet radiation, in particular for use in food processing or water treatment, comprising an UV emitter with an emitter tube made of quartz glass or an UV emitter surrounded by a cylinder-shaped cladding tube made of quartz glass with an emitter tube made of quartz glass, **characterised in that** a dirt-repelling coating produced through the use of silicon dioxide or titanium dioxide nanoparticles is applied onto the emitter tube and/or the cladding tube.

2. Emitter unit according to claim 1, **characterised in that** the coating comprises no organic substances.

3. Emitter unit according to claim 1 or 2, **characterised in that** the coating comprises a surface with a mean roughness Ra of less than 0.05 µm.

4. Emitter unit according to any one of the preceding claims, **characterised in that** the silicon dioxide nanoparticles comprise a mean particle size in the range of 1 nm to 75 nm.

5. Emitter unit according to any one of the preceding claims, **characterised in that** the titanium dioxide nanoparticles comprise a mean particle size between 1 nm and 80 nm.

6. Emitter unit according to any one of the preceding claims, **characterised in that** the mean layer thickness of the coating is between 60 nm and 150 nm.

7. Emitter unit according to any one of the preceding claims, **characterised in that** the emitter tube and/or the cladding tube comprises a surface of a mean roughness Ra in the range between 0.01 µm and 1 µm onto which the coating is applied.

8. Emitter unit according to any one of the preceding claims, **characterised in that** the emitter tube comprises an emission surface that is completely provided with the coating.

9. Method for producing an emitter unit according to claim 1 with a coated emitter tube made of quartz glass and/or a coated cladding tube made of quartz glass by providing the emitter tube or the cladding tube and generating a coating on at least a part of the external wall, comprising the following procedural steps:
(c) applying an alcoholic dispersion of silicon dioxide or titanium dioxide nanoparticles onto the external wall while forming a dispersion layer, whereby the alcoholic dispersion comprises 20% by volume to 60% by volume ethanol, each relative to the volume of the dispersion,
(d) curing the dispersion layer while forming the coating.

10. Method according to claim 9, **characterised in that** the alcoholic dispersion comprises 0.25% by volume to 1.5% by volume 2-butanone.

## Revendications

1. Module émetteur de rayonnement pour la génération de rayonnement ultraviolet, notamment pour l'utilisation lors de la transformation d'aliments ou lors de la préparation d'eau, comprenant un émetteur de rayonnement UV avec un tube d'émetteur de rayonnement en verre de quartz ou un émetteur de rayonnement UV entouré par un tube d'enveloppe cylindrique en verre de quartz avec un tube d'émetteur de rayonnement en verre de quartz, **caractérisé en ce qu'**un revêtement antisalissant est appliqué sur le tube d'émetteur de rayonnement et/ou le tube d'enveloppe, lequel est généré en utilisant des nanoparticules de dioxyde de silicium ou de dioxyde de titane.

2. Module émetteur de rayonnement selon la revendication 1, **caractérisé en ce que** le revêtement ne comprend aucune substance organique.

3. Module émetteur de rayonnement selon la revendication 1 ou 2, **caractérisé en ce que** le revêtement présente une surface avec une rugosité moyenne Ra de moins de 0,05 µm.

4. Module émetteur de rayonnement selon une des revendications précédentes, **caractérisé en ce que** les nanoparticules de dioxyde de silicium présentent une taille particulaire moyenne dans la région de 1 nm à 75 nm.

5. Module émetteur de rayonnement selon une des revendications précédentes, **caractérisé en ce que** les nanoparticules de dioxyde de titane présentent une taille particulaire moyenne comprise entre 1 nm et 80 nm.

6. Module émetteur de rayonnement selon une des revendications précédentes, **caractérisé en ce que** l'épaisseur de couche moyenne du revêtement est comprise entre 60 nm et 150 nm.

7. Module émetteur de rayonnement selon une des revendications précédentes, **caractérisé en ce que** le tube d'émetteur de rayonnement et/ou le tube d'enveloppe présente une surface avec une rugosité moyenne Rₐ dans la région comprise entre 0,01 µm et 1 µm sur laquelle le revêtement est appliqué.

8. Module émetteur de rayonnement selon une des revendications précédentes, **caractérisé en ce que** le tube d'émetteur de rayonnement présente une face de rayonnement qui est entièrement pourvue du revêtement.

9. Procédé de fabrication d'un module émetteur de rayonnement selon la revendication 1 avec un tube d'émetteur de rayonnement enduit en verre de quartz et/ou un tube d'enveloppe enduit en verre de quartz par mise à disposition du tube d'émetteur de rayonnement ou du tube d'enveloppe et génération d'un revêtement sur au moins une partie de la paroi externe, comprenant les étapes de procédé suivantes :
(c) application d'une dispersion alcoolique de nanoparticules de dioxyde de silicium ou de dioxyde de titane sur la paroi externe en formant une couche de dispersion, dans lequel la dispersion alcoolique comprend 20 % en vol. à 60 % en vol. d'éthanol, à chaque fois par rapport au volume de la dispersion,
(d) durcissement de la couche de dispersion en formant le revêtement.

10. Procédé selon la revendication 9, **caractérisé en ce que** la dispersion alcoolique comprend 0,25 % en vol. à 1,5 % en vol. de 2-butanone.
